# EUROPÄISCHE PATENTSCHRIFT

(11) **EP 3 782 686 B1**
(45) Veröffentlichungstag und Bekanntmachung des Hinweises auf die Patenterteilung: **05.07.2023**
(21) Anmeldenummer: 20000263.2
(22) Anmeldetag: 22.07.2020
(51) Int. Cl.: A61M 16/10, A61M 16/00

(54) **SYSTEM UND BEATMUNGSGERÄT ZUR NICHTINVASIVEN INFEKTIONSERKENNUNG WÄHREND DER BEATMUNG**
SYSTEM AND VENTILATOR FOR NON-INVASIVE DETECTION OF INFECTION DURING VENTILATION
SYSTÈME ET DISPOSITIF RESPIRATOIRE DESTINÉS À LA DÉTECTION NON INVASIVE D'UNE INFECTION LORS DE RESPIRATION

(30) Priorität: 20.08.2019 DE 102019005846
(43) Veröffentlichungstag der Anmeldung: 24.02.2021
(73) Patentinhaber: Löwenstein Medical Technology S.A., 2557 Luxembourg (LU)
(72) Erfinder: Schwaibold, Matthias, 76228 Karlsruhe (DE)
(74) Vertreter: Marx, Thomas

(56) Entgegenhaltungen:
- WO-A1-2015/020536
- WO-A2-03/075745
- US-A1- 2003 176 804
- US-A1- 2018 082 033

## Beschreibung

Die Erfindung betrifft ein System zur nichtinvasiven Infektionserkennung während der Beatmung, mit zumindest einem Sensor, der zur Infektionserkennung ausgebildet ist und einem Beatmungsgerät.

Bei Patienten die im häuslichen Umfeld oder in der Klinik beatmet werden, können Infekte nur schwer, oder oft erst spät erkannt werden. Insbesondere bei chronisch stabilen Lungenerkrankungen (COPD, Asthma, NMD) sind Atemwegsinfekte häufig Auslöser für eine Dekompensation / Exazerbation, die eine zusätzliche Behandlung erforderlich macht.

Infektionen können über einen Anstieg der Körpertemperatur identifiziert werden.

Es gibt verschiedene Einrichtungen und Verfahren zur Messung der Körpertemperatur von Menschen. Bei invasiven Messverfahren werden Temperatursensoren über Körperöffnungen in den eingebracht und über das Signal der Temperatursensoren auf die Kerntemperatur rückgeschlossen.

Derartige Messmethoden sind jedoch anfällig für Artefakte und werden dauerhaft nicht gut toleriert.

Aus der WO 2015/020536 A1 ist ein Beatmungssystem bekannt, welches eine Kontrolle unter anderem über ein mobiles Endgerät erlaubt. Über Sensoren ist zudem eine Kontrolle der Schlaf-/Wachphase und eine optionale darauf angepasste Beatmung ermöglicht.

Die WO 03/075745 A2 präsentiert eine Methode, die Ausatemluft auf mögliche Infektion, insbesondere über biochemische Analysen, zu untersuchen.

Ein System zur Steuerung von Beatmungsgeräten über ein Netzwerk wird in der US 2018/0082033 A1 präsentiert. Zudem wird eine Steuerung der Beatmung anhand eines Aktivitätslevels des Patienten vorgeschlagen.

Aus der US 2003/0176804 A1 ist ein Verfahren und ein System zu entnehmen, welches die Atemluft eines Patienten insbesondere hinsichtlich Anästhesiegasen untersucht um die Tiefe der Anästhesie zu bestimmen. Hierbei wird auch die Möglichkeit eröffnet, das Atemgas auf weitere Biomarker zu überprüfen.

Gerade während der Beatmung ist die Messung der Temperatur mit herkömmlichen Methoden schwer vorzunehmen.

Die Zielsetzung der vorliegenden Erfindung ist eine frühzeitige Erkennung eines Atemwegsinfektes während der Beatmung, bevor es zu einer schweren Exazerbation kommt.

Die Aufgabe der vorliegenden Erfindung besteht daher in der Schaffung eines Systems zur nichtinvasiven Infektionserkennung mit zumindest einem Sensor, welcher möglichst einfach und kostengünstig aufgebaut ist, und in das Beatmungssystem integriert ist und zur Infektionserkennung ausgebildet ist.

Gelöst wird die erfindungsgemäße Aufgabe durch ein System zur nichtinvasiven Infektionserkennung während der Beatmung mit zumindest einem Sensor, der zur Infektionserkennung ausgebildet ist, einem Beatmungsgerät, einer Schnittstelle zwischen Beatmungsgerät und Sensor, einer Verarbeitungseinheit, einem Datenspeicher, einem User Interface, einer Schnittstelle zu einem Remote Alarm, einem Remote Alarm, einem Beatmungsschlauch mit einem Patienteninterface und zumindest einem Atemgassensor, wobei das Beatmungsgerät eingerichtet und ausgebildet ist mittels des Atemgassensors den Druck und/oder Fluss des Atemgases zu identifizieren und unter Druck stehendes Atemgas zu fördern, wobei das Beatmungsgerät dazu ausgebildet ist eine Atemfrequenz zu bestimmen und wobei das System eingerichtet ist zumindest phasenweise während der Beatmung mittels des Sensors und der Verarbeitungseinheit ein Sensorsignal zu ermitteln und zu überprüfen, ob das Sensorsignal repräsentativ für eine Infektion ist dadurch gekennzeichnet, dass die Atemfrequenz mit zumindest einem weiteren Sensorsignal verknüpft wird und anhand dieser verknüpften Sensorsignale eine mögliche Infektion beziehungsweise die Wahrscheinlichkeit einer Infektion ermittelt wird.

Das Beatmungsgerät ist bevorzugt eingerichtet und ausgebildet ist mittels des Atemgassensors Phasen der Inspiration und Exspiration zu identifizieren und Atemgas während der Inspiration und Exspiration zu fördern. Das System ist bevorzugt eingerichtet zumindest phasenweise während der Inspiration und Exspiration mittels des Sensors und der Verarbeitungseinheit ein Sensorsignal zu ermitteln und zu überprüfen, ob das Sensorsignal repräsentativ für die Körpertemperatur oder einen Infekt ist und solche Sensorsignale zu verarbeiten und/oder zu speichern und mit hinterlegen Regeln oder Schwellwerten zu vergleichen.

Wesentlich an der vorliegenden Erfindung ist alternativ auch, dass mit dem zumindest einen Sensor eine Sendeeinrichtung zum Übertragen der gemessenen Sensorwerte verbunden ist. Erfindungsgemäß ist der Sensor 2 zur Erkennung einer Infektion oder Entzündung ausgebildet. Der Sensor ist ausgebildet zur Erkennung von Parametern oder Messwerten die indikativ für eine Infektion oder Entzündung sind.

Der Sensor kann dazu als Temperatursensor oder Aktivitätssensor als alternativ ausgebildet sein zur Erkennung von Keimen in der Luft, Anstieg der mittleren Herzfrequenz, Veränderung der Hautimpedanz, rasche Gewichtsabnahme, oder weiteren Parametern die indikativ für eine Infektion oder Entzündung sind.

Nach einer vorteilhaften Ausführungsform ist das System auch dadurch gekennzeichnet, dass zumindest ein Temperatursensor durch einen Thermistor gebildet ist.

Nach einer alternativen vorteilhaften Ausführungsform ist das System auch dadurch gekennzeichnet, dass zumindest ein Temperatursensor durch einen optischen Sensor gebildet ist.

Nach einem Aspekt der Lehre der Erfindung wird vorgeschlagen, dass zumindest ein Temperatursensor in dem Schlauch oder dem Patienteninterface zur Messung der Temperatur der Inspirations- und/oder Exspirationsströmung vorgesehen ist.

Nach einem weiteren Aspekt der Lehre der Erfindung wird vorgeschlagen, dass zumindest ein Temperatursensor dem Körper oder Haut des Patienten benachbart angeordnet ist

Nach einem ergänzenden Aspekt der Lehre der Erfindung wird vorgeschlagen, dass zumindest ein Temperatursensor im Bereich des Patienteninterface vorgesehen ist und so angeordnet ist, dass der Sensor dem Körper oder Haut des Patienten benachbart angeordnet ist, beispielsweise montiert durch eine Klebefläche oder einen Magneten oder einen Clip an der Stirnstütze des Patienteninterface oder an der Bänderung des Patienteninterface oder am Wulst des Patienteninterface oder am Körper des Patienteninterface oder an einem Finger, der Stirn oder dem Ohrläppchen des Patienten.

Nach einem alternativen Aspekt der Lehre der Erfindung wird vorgeschlagen, dass zumindest ein Temperatursensor dem Körper oder Haut des Patienten entfernt angeordnet ist beispielsweise in Form einer Wärmebildkamera.

Nach einer weiteren vorteilhaften Ausführungsform ist vorgehen, dass ergänzend zu dem Temperatursensor auch ein Aktivitäts-Sensor vorgesehen ist.

Nach einer alternativen oder ergänzenden Ausführungsform ist vorgehen, dass der Sensor als ein Aktivitäts-Sensor ausgebildet ist, der ausgebildet sein kann, eine Infektion anhand der Abnahme der körperlichen Aktivität, im Vergleich zu einem früheren Vergleichszeitraum, zu erkennen.

Nach einer ergänzenden vorteilhaften Ausführungsform ist vorgehen, dass die Schnittstelle zwischen dem Beatmungsgerät und dem Temperatursensor entweder nur Daten (Temperatursensor hat dann eine eigene Energiequelle) oder Daten und Energieversorgung für Temperatursensor leitet.

Beispielsweise ist das System auch dadurch gekennzeichnet, dass die Schnittstelle als Kabel beispielsweise USB, IC, seriell, Lichtleiter, LAN, ESATA, ...ausgeführt ist.

Ergänzend oder alternativ ist das System auch dadurch gekennzeichnet, dass die Schnittstelle drahtlos beispielsweise als Bluetooth, WIFI, Low-Power-Standard ausgeführt ist.

Ergänzend oder alternativ ist das System auch dadurch gekennzeichnet, dass die Übertragung von Sensordaten über die Schnittstelle mindestens täglich erfolgt vorzugsweise mindestens stündlich, besonders bevorzugt mindestens 1x bis 60x pro Minute.

Vorteilhaft ist das System auch dadurch gekennzeichnet, dass die Verarbeitungseinheit ausgebildet und eingerichtet ist zu erkennen, ob ein Sensorsignal vorhanden ist und ob ein Sensorsignal ein Temperatursignal repräsentiert.

Vorteilhaft ist das System auch dadurch gekennzeichnet, dass die Verarbeitungseinheit das Sensorsignal vergleicht mit einem hinterlegten Wertebereich, der typische Köpertemperatursignale repräsentiert beispielsweise im Bereich zwischen 33 und 45 Grad Celsius, bevorzugt zwischen 35 und 44 Grad Celsius.

Nach einer weiteren vorteilhaften Ausführungsform ist vorgehen, dass die Verarbeitungseinheit das Sensorsignal auf wiederkehrende maximale und minimale Temperatursignale überprüft und zumindest die maximalen Temperatursignale als Körpertemperatursignale wertet.

Nach einer vorteilhaften Ausführungsform ist vorgehen, dass die Verarbeitungseinheit das Sensorsignal des Temperatursensors mit dem Sensorsignal des Aktivitätssensors abgleicht. Das System ist beispielsweise alternativ oder ergänzend auch dadurch gekennzeichnet, dass die Verarbeitungseinheit das Sensorsignal des Temperatursensors mit dem Sensorsignal des Aktivitätssensors abgleicht und solche Sensorsignale des Temperatursensors als nächtliche Körpertemperatursignale verarbeitet, die zeitgleich mit einem geringen Sensorsignal des Aktivitätssensors über einen Zeitraum von zumindest 3 Stunden registriert werden.

Das System ist beispielsweise alternativ oder ergänzend auch dadurch gekennzeichnet, dass die Verarbeitungseinheit ausgebildet und eingerichtet ist das Temperatursignal aufzubereiten beispielsweise durch Umrechnung der Einheiten, Glättung, ...und in dem Datenspeicher abzuspeichern. Die Umrechnung von Einheiten kann zum Beispiel eine Umrechnung von Sensorsignalen, welche analog in Form einer Spannung oder digital als Bitwert vorliegen und in eine mit der gemessenen physiologischen Größe stehenden Einheit umgerechnet werden. Im Falle einer gemessenen Temperatur kann das zum Beispiel Grad Celsius oder Grad Fahrenheit oder eine ähnliche, mit der Temperatur im Zusammenhang stehende Größe bzw. Einheit sein. Eine Glättung der Einheiten kann beispielsweise durch eine Mittelung oder eine gewichtete Mittelung der Signale über einen Zeitraum erfolgen. Auch die Entfernung von Ausreißersignalen kann als Maßnahme zur Glättung herangezogen werden. Als Ausreißersignale können beispielsweise einzeln vorkommende Signale oder Werte betrachtet werden, welche nicht in einem plausiblen Bereich einer Körpertemperatur liegen oder der Wert zu weit von den vorherigen Messwerten abweicht. Treten Ausreißersignale häufiger und/oder zeitlich direkt aufeinander folgend auf, können diese auch als nicht-Ausreißersignale gewertet werden und eventuell einen entsprechenden Alarm auslöst.

Das System ist alternativ oder ergänzend auch dadurch gekennzeichnet, dass die Verarbeitungseinheit ausgebildet und eingerichtet ist das Temperatursignal mit hinterlegten Alarmschwellen abzugleichen wobei die Alarmschwellen eine zu hohe Temperatur oder niedrige Temperatur oder den Verlust der Datenverbindung oder ein Abfallen des Sensors oder einen Ablauf der Lebensdauer des Sensors oder zu niedrigem Batterie-Stand des Sensors repräsentieren.

Das System ist beispielsweise alternativ oder ergänzend auch dadurch gekennzeichnet, dass die Verarbeitungseinheit ausgebildet und eingerichtet ist das Temperatursignal mit hinterlegten Alarmschwellen abzugleichen und bei Überschreiten einer Alarmschwelle für erhöhte Körpertemperatur die Verarbeitungseinheit ausgebildet und eingerichtet ist zumindest einen Beatmungsparameter des Beatmungsgerätes so zu steuern, dass eine Beatmung resultiert, die der erhöhten Körpertemperatur angepasst ist, sodass eine optimierte Entlastung oder Unterstützung für den Patienten gewährleistet ist.

Vorteilhaft ist das System auch dadurch gekennzeichnet, dass die Verarbeitungseinheit ausgebildet und eingerichtet ist das Temperatursignal mit hinterlegten Alarmschwellen abzugleichen und bei Überschreiten einer Alarmschwelle für eine erhöhte oder erniedrigte Körpertemperatur (Temperaturalarm) die Verarbeitungseinheit ausgebildet und eingerichtet ist zumindest eine Schwelle für einen Beatmungsparameteralarm (Beatmungsalarm) zu verändern. Beispielsweise wird der Beatmungsalarm bei erhöhter Körpertemperatur sensitiver.

Erfindungsgemäß ist das System auch dadurch gekennzeichnet, dass die Verarbeitungseinheit ausgebildet und eingerichtet ist das Temperatursignal mit hinterlegten Alarmschwellen abzugleichen und bei Überschreiten einer Alarmschwelle für eine erhöhte oder erniedrigte Körpertemperatur (Temperaturalarm) die Verarbeitungseinheit ausgebildet und eingerichtet ist automatisiert eine Rückmeldung des Patienten anzufordern, wobei die Rückmeldung eine digitale Antwort des Patienten ist, die eine aktuelle Befindlichkeit repräsentiert.

Vorteilhaft ist das System auch dadurch gekennzeichnet, dass der Patient seine Antwort über die Benutzeroberfläche eingibt.

Ein Aspekt der Erfindung sieht vor, dass die Verarbeitungseinheit ausgebildet und eingerichtet ist das Temperatursignal mit hinterlegten Alarmschwellen abzugleichen und bei Überschreiten einer Alarmschwelle für eine erhöhte oder erniedrigte Körpertemperatur (Temperaturalarm) die Verarbeitungseinheit ausgebildet und eingerichtet ist über die Benutzeroberfläche oder einen Lautsprecher Handlungsempfehlungen an den Patienten auszugeben.

Vorteilhaft ist das System auch dadurch gekennzeichnet, dass die Verarbeitungseinheit ausgebildet und eingerichtet ist Statistiken, beispielsweise enthaltend Max / Min / Mittelwerte / Anteil mit Wert > x für definierte Zeitintervalle, z. B. pro Stunde oder pro Tag, zu bilden und im Datenspeicher abzulegen. Auch eine Alarmauslösung kann auf diesen Statistiken basierend erzeugt werden. Liegt der Wert der Temperatur für eine gewisse Zeit über einem Wert, so kann dies als Auslöser für den Alarm dienen. Hier könne auch Abstufungen eingeführt werden. So kann beispielsweise der Temperaturwert für eine Zeitdauer, z.B. über eine Stunde, bei über 38 Grad Celsius liegen, ohne dass ein Alarm ausgelöst wird, für höhere Temperaturwerte, z.B. über 40 Grad Celsius, weitaus wenig, z.B. bereits bei einer Minute oder weniger.

Schließlich ist nach einem weiteren Aspekt der Lehre die Verarbeitungseinheit ausgebildet und eingerichtet Signale des Temperatursensors und Signale des Atemgassensors und Signale des Aktivitäts-Sensors zu verarbeiten und Signale des Temperatursensors zu plausibilisieren durch einen Vergleich mit Signalen des Atemgassensors und/oder Signalen des Aktivitäts-Sensors.

Insbesondere kann das System auch dadurch gekennzeichnet sein, dass der Datenspeicher ausgebildet und eingerichtet ist den Verlauf des Temperatursignals und vorzugsweise auch die berechneten Statistiken und aufgetretenen Alarme abrufbar aufzuzeichnen.

Das System ist alternativ auch dadurch gekennzeichnet, dass das User Interface beispielsweise als Display, LEDs, Lautsprecher, Projektionsfläche, ausgebildet und eingerichtet ist das Temperatursignal als Temperatur darzustellen und optional auch Statistiken, Alarme, Verläufe darzustellen.

Das System ist alternativ oder ergänzend auch dadurch gekennzeichnet, dass das User Interface ausgebildet und eingerichtet ist ein Rückmeldung zu generieren, ob ein Temperatursensor korrekt angeschlossen ist und Werte liefert oder nicht, bevorzugt in Form einer grafischen und akustischen Ausgabe, z. B. Warnton im Falle eines Alarms.

Das System ist alternativ oder ergänzend auch dadurch gekennzeichnet, dass die Schnittstelle zum Remote Alarm ausgebildet und eingerichtet ist entweder nur Daten (Remote Alarm hat eine eigene Energiequelle) oder Daten und Energie in Bezug auf den Remote Alarm zu leiten. Das System ist alternativ oder ergänzend auch dadurch gekennzeichnet, dass die Schnittstelle zum Remote Alarm als Kabel (USB, IC, seriell, Lichtleiter, LAN, ESATA, I2C, Analogleitungen mit variablem Spannungspegel, ...) oder drahtlos (Bluetooth, WIFI, Low-Power-Standard, GSM, UMTS, LTE, NB-IOT, Lora, Sigfox, G Mobilfunk,) ausgebildet ist. Das System ist alternativ oder ergänzend auch dadurch gekennzeichnet, dass die Schnittstelle zum Remote Alarm bevorzugt mindestens teilweise aus einem Netzwerk, z. B. Kliniknetzwerk, Internet, VPN-Netzwerk besteht oder in ein Netzwerk eingebunden ist.

Das System ist alternativ oder ergänzend auch dadurch gekennzeichnet, dass die Schnittstelle zum Remote Alarm zur mindestens täglichen, vorzugsweise mindestens stündlich, bevorzugt mindestens 1x bis 60x pro Minute Übertragung eingerichtet und ausgebildet ist; vorzugsweise auch getriggert durch das Auftreten eines Alarms.

Vorteilhaft ist das System auch dadurch gekennzeichnet, dass der Remote Alarm ausgebildet und eingerichtet ist, beispielsweise auf einem Computerbildschirm, z. B. im Browser bei webbasiertem Telemonitoring oder angesteuert durch eine lokale Software, die mit dem Beatmungsgerät kommunizieren kann oder auf einem Handheld-Gerät wie Smartphone, Notebook oder Tablet, ebenfalls entweder im Browser oder angesteuert durch eine lokale SW (Software) oder durch einen akustischen Signalgeber (Schwesternruf) oder in Form einer Nachricht, z. B. SMS, Messenger, Email ausgeführt zu werden.

Weiter vorteilhaft ist das System auch dadurch gekennzeichnet, dass das System einen Anfeuchter umfasst, der eingerichtet und ausgebildet ist Atemgas zu erwärmen und anzufeuchten, wobei Körpertemperatursignale des Temperatursensors zumindest zeitweise oder teilweise für die Steuerung des Anfeuchters berücksichtigt werden, um das Atemgas entsprechend dem Köpertemperatursignal zu erwärmen und anzufeuchten.

Ergänzend vorteilhaft ist das System auch dadurch gekennzeichnet, dass das System zudem einen Temperatursensor umfasst, der die Umgebungstemperatur misst und die Verarbeitungseinheit ausgebildet und eingerichtet ist das Temperatursignal des Sensors mit dem Temperatursignal des Sensors abzugleichen.

Beispielsweise weiter vorteilhaft ist das System auch dadurch gekennzeichnet, dass das System zudem einen Temperatursensor umfasst, der die Umgebungstemperatur misst und die Verarbeitungseinheit ausgebildet und eingerichtet bei Überschreiten einer Alarmschwelle für eine erhöhte oder erniedrigte Umgebungstemperatur einen Alarm auszugeben, beispielsweise einen Remotealarm.

Bevorzugt ist das System auch dadurch gekennzeichnet, dass das System zudem einen Temperatursensor, der die Umgebungstemperatur misst, und eine Klimatisierungseinheit für die Umgebungstemperatur umfasst und die Verarbeitungseinheit ausgebildet und eingerichtet bei Überschreiten einer Alarmschwelle für eine erhöhte oder erniedrigte Umgebungstemperatur Klimatisierungseinheit so zu steuern, dass die Umgebungstemperatur angepasst wird. Das Beatmungsgerät ist ein klinisches Beatmungsgerät oder ein Beatmungsgerät für die häusliche Beatmung oder CPAP- oder Bilevel-Therapie oder ein Highflow-Beatmungsgerät oder ein beliebiges anderes Beatmungsgerät.

Das System ist alternativ oder ergänzend dadurch gekennzeichnet, dass es einen Sensor zur Aktivitätserkennung umfasst. Ein solcher Sensor kann zum Beispiel ein Beschleunigungssensor oder auch ein Bewegungssensor sein. Dieser Sensor wird am Körper (zum Beispiel Handgelenk und/oder Brust) angebracht und/oder ist ein Sensor in einem mobilen Gerät mit einer Datenschnittstelle (zum Beispiel Smartphone) und/oder auch ein Sensor in einem Bett, über welchen die tägliche Liegedauer erfasst werden kann. Ein solcher Sensor zu Aktivitätserkennung lässt sich auch mit weiteren Sensoren, zum Beispiel zur Temperaturüberwachung, kombinieren. In manchen Ausfuhrungsformen ist der Aktivitätssensor auch fest in dem Raum installiert, in welchem beispielsweise das Beatmungsgerät genutzt wird oder der Patient behandelt wird.

Das System kann beispielsweise basierend auf den Signalen des Aktivitätssensors die Aktivität des Patienten auswerten und gegebenenfalls einen Alarm auslösen. So kann beispielsweise ein Alarm ausgelöst werden, wenn die Aktivität des Patienten über einen bestimmten Zeitraum sehr niedrig und/oder nicht vorhanden ist. Beispielsweise ist der Aktivitätssensor kombiniert mit einer Wärmebildkamera. Auch ist das System in manchen Ausführungsformen dazu in der Lage die durch den Aktivitätssensor registrierte Aktivität mit Aktivitätsaufzeichnungen des Patienten zu vergleichen um eine erhöhte oder verringerte Aktivität festzustellen. Zudem ist das System in manchen Ausführungsformen dazu eingerichtet, die Aktivität anhand anderer Sensorsignale, beispielsweise Temperatur- und/oder Atemgassensoren, sowie eventuellen Analysen dieser Sensorsignale, zu bewerten.

Alternativ oder ergänzend ist das System auch dadurch gekennzeichnet, dass das System einen Sensor zur Pulsoximetrie umfasst, welcher unter anderem die Sauerstoffsättigung des Blutes beispielsweise über Lichtabsorption und/oder Lichtremission/-reflexion bestimmen kann. Aus den Messwerten des Sensors zur Pulsoxymetrie kann die Verarbeitungseinheit des Systems zum Beispiel Rückschlüsse auf den Gesundheitsstand des Patienten ziehen. So wird in der Regel eine Sauerstoffsättigung zwischen 95% und 100% als ausreichend angesehen, während beispielsweise Werte von unter 85% Sauerstoffsättigung ein gesundheitliches Problem indizieren können. Ein solcher Sensor ist in der Lage die Sauerstoffsättigung und auch die Pulsfrequenz zu messen. Mit einem Sensor zur Pulsoximetrie kann auch ein Temperatursensor kombiniert werden. Neben Hinweisen bezüglich etwaiger Erkrankungen oder bezüglich der Befindlichkeit des Nutzers bzw. des Patienten, können in manchen Ausführungsformen auch Rückschlüsse auf die Beatmungsqualität aus den Signalen der Pulsoximetrie gezogen werden. In manchen Ausführungsformen werden basierend auf der Sauerstoffsättigung und, optional, der Temperatur oder anderen Sensorsignalen, die Beatmungseinstellungen bzw. -parameter angepasst. Diese Anpassung kann unter anderem auch automatisch erfolgen.

Zusätzlich oder alternativ umfasst das System zumindest einen Sensor zur Analyse der Ausatemluft bezüglich allgemeiner Biomarker zum Beispiel für Entzündungsreaktionen oder auch für spezifische Krankheitserreger. Optional ist auch eine Analyse der Temperatur über die Ausatemluft möglich. Dazu ist in manchen Ausführungsformen des Systems der Sensor oder die Sensoren im Bereich des Patienteninterface eingerichtet. Der Sensor zu Analyse der Ausatemluft kann beispielsweise auch im Beatmungsgerät oder zwischen dem Beatmungsgerät und dem Patienteninterface angeordnet sein, soweit die Ausatemluft in Richtung Beatmungsgerät geleitet wird. Auch kann der Sensor zur Analyse der Ausatemluft entfernt vom Beatmungsgerät und vorteilhafterweise in Verbindung mit dem Patienteninterface angeordnet sein.

Das System ist alternativ oder ergänzend dadurch gekennzeichnet, dass das System zumindest eine Einrichtung bzw. zumindest einen Sensor umfasst, um eine "Point-of-Care" Analyse bestimmter Körperextrakte, -ausscheidungen und/oder -flüssigkeiten durchzuführen. Diese Extrakte, Flüssigkeiten und/oder Ausscheidungen können dabei beispielsweise Urin, Blut, Speichel, Auswurf, Haare, Nägel und anderes sein. Insbesondere eine Analyse hinsichtlich Temperatur, Farbspektrum, Flüssigkeitsgehalt oder bestimmter Inhaltsstoffe untersucht werden. In manchen Ausführungsformen kann das Beatmungsgerät beziehungsweise die Beatmungseinstellungen automatisch auf die Ergebnisse der Analyse der Extrakte/Flüssigkeiten/Ausscheidungen eingestellt werden. Eine Analyse der Körperextrakte, -ausscheidungen und/oder -flüssigkeiten kann beispielsweise durch die Analyse von Teststreifen durch einen entsprechenden Sensor erfolgen. Ein solcher Sensor kann Beispielsweise ausgebildet sein, die Verfärbung einzelner Testfelder eines Teststreifens, welcher zuvor mit den Körperextrakten, -ausscheidungen und/oder -flüssigkeiten in Kontakt gebracht wurde, zu erkennen. Die Verarbeitungseinheit des Systems kann basierend auf der Erkennung der Verfärbungen beispielsweise Infektionen oder Hinweise auf Infektionen erkennen beziehungsweise prüfen ob die Verfärbung des Teststreifens repräsentativ für eine Infektion ist. In manchen Ausführungsformen ist der zumindest eine Sensor ein biochemischer Sensor, welcher ausgebildet und eingerichtet ist, Körperextrakten, - ausscheidungen und/oder -flüssigkeiten direkt auf bestimmte Inhaltsstoffe zu analysieren oder zu messen. In manchen Ausfuhrungsformen ist das System beispielsweise auch so eingerichtet, dass Körperextrakte, -ausscheidungen und/oder -flüssigkeiten direkt durch das System entnommen und/oder aufgefangen werden können. Beispielsweise kann eine entsprechende Auffangeinrichtung im Bereich des Patienteninterfaces oder des Beatmungsschlauchs zum Auffangen von Auswurf des Patienten - z.B. durch Husten - angebracht sein, welche zudem über einen entsprechenden Sensor oder mehrere Sensoren verfügt, welche zur Infektionserkennung eingerichtet sind. Wie zuvor bereits unter anderem im Zusammenhang mit einem Temperatursensor beschrieben, können auf Basis der Sensorsignale bestimmte Aktionen eingeleitet werden, wie zum Beispiel Hinweise an den Patienten, Generierung eines Alarmsignals bzw. Auslösen eines Signals, Speicherung/Übermittlung der Daten per Telemonitoring, etc..

Die Signale der vorangehend beschriebenen Sensoren können in verschiedenen alternativen oder sich ergänzenden Ausführungsformen des Systems ausgewertet werden und das Über- oder Unterschreiten definierter Schwellwerte als eine Infektion erkennen. Die Verarbeitungseinheit des Beatmungsgeräts ist dabei so eingerichtet und ausgebildet, dass erkannt werden kann, ob ein Sensor angeschlossen ist und um welche Art von Sensor es sich handelt bzw. welche Art von Sensordaten übermittelt werden. Die Verarbeitungseinheit ist zudem in manchen Ausführungsformen dazu eingerichtet und ausgebildet, die Sensorsignale mit Alarmschwellen zu vergleichen und darauf basierend eine Abfrage der Befindlichkeit Patienten zu erzeugen und/oder entsprechende Handlungsempfehlungen an den Patienten über eine Benutzeroberfläche auszugeben. Alternativ oder ergänzend kann durch das Über- oder Unterschreiten der Alarmschwellen auch entsprechende Daten an einen Remote-Alarm gesendet werden.

Unabhängig von der Art des Sensors bzw. der Sensoren wird in manchen Ausführungsformen zu jedem Sensorsignal ein Index-Wert berechnet wird, welcher zumindest enthält ob das Sensorsignal repräsentativ für eine Infektion ist. Die jeweiligen Index-Werte der einzelnen Sensorsignale können beispielsweise aufaddiert oder über einen anderen Algorithmus zusammengerechnet werden um einen Gesamt-Index zu berechnen. Für diesen Gesamt-Index können weiter Schwellenwerte hinterlegt werden, bei deren Überschreitung verschiedene Aktionen ausgelöst werden, wie zum Beispiel zunächst ein einfacher Hinweis an den Patienten und verschiedene Alarmstufen. In manchen Ausführungsformen kann auch eine Datenübertragung z.B. per Telemonitoring eingerichtet sein.

In manchen beispielhaften Ausführungsformen können verschiedene Regeln, z.B. sogenannte "Fuzzy-Regeln" definiert werden, wonach die Sensorsignale miteinander verknüpft werden und anhand dieser verknüpften Sensorsignalen eine mögliche Infektion beziehungsweise die Wahrscheinlichkeit einer Infektion ermittelt wird. Erfindungsgemäß werden die Sensorsignale mit der über das Beatmungsgerät bestimmten Atemfrequenz verknüpft. So kann beispielweise eine stark erhöhte Atemfrequenz in Kombination mit zumindest einem von einem als Normalbereich definierten Wert abweichenden Sensorsignal deutlich auf eine Infektion hinweisen.

Weiter können in manchen Ausführungsformen Sensorsignale auch über einen Entscheidungsbaum miteinander verknüpft werden um eine Infektion oder die Wahrscheinlichkeit einer Infektion zu bestimmen. So können verschiedenen Sensorsignalen beispielsweise unterschiedlich stark gewichtet werden. Auch kann eine Verzweigung der einzelnen Sensorsignale in einem Entscheidungsbaum beispielsweise durch ein Machine-Learning-Verfahren erstellt werden.

In manchen Ausführungsformen wird Zuschaltung von weiteren Sensoren entsprechend eines sensitiven Vor-Verdachts einer Infektion ausgelöst werden oder der Patient! Anwender wird aufgefordert weitere Sensoren aufgrund dieses Vorverdachts zuzuschalten. Beispielsweise kann ein Anfangsverdacht auf eine Infektion durch eine leicht, zum Beispiel mindestens durchschnittlich 2 Atemzüge pro Minute, erhöhte Atemfrequenz ausgelöst werden. Dieser Anfangsverdacht kann zum Beispiel dann einen Hinweis an den Patienten verursachen, dass weitere Sensoren, z.B. Pulsoxymeter oder Thermometer, verwendet werden sollen. Weitere Indikatoren zum Vorliegen einer Infektion können beispielsweise auch aus der Nutzungsdauer des Beatmungsgeräts oder allgemein ungewöhnliche Schwankungen in Atemfrequenz und/oder -volumen bestimmt werden.

Die Unteransprüche und auch die Beschreibung betreffen verschiedene voneinander unabhängige, vorteilhafte Weiterbildungen der vorliegenden Erfindung, deren Merkmale vom Fachmann im Rahmen des technisch Sinnvollen frei miteinander kombiniert werden können. Dies gilt insbesondere auch über die Grenzen der verschiedenen Anspruchskategorien hinaus.

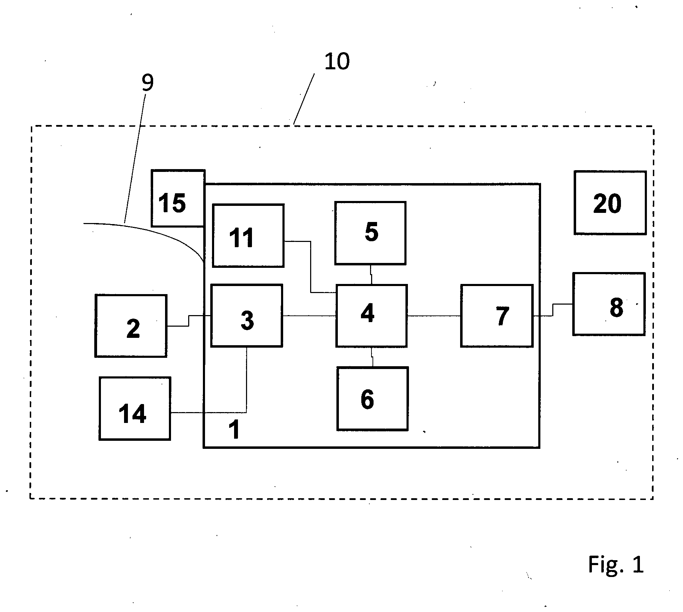

Figur 1 zeigt ein System 10 zur nichtinvasiven Messung der Körpertemperatur während der Beatmung mit zumindest einem Sensor 2, einem Beatmungsgerät 1, einer Schnittstelle 3 zwischen Beatmungsgerät und Temperatursensor, einer Verarbeitungseinheit 4, einem Datenspeicher 5, einem User Interface 6, einer Schnittstelle 7 zu einem Remote Alarm, einem Remote Alarm 8, einem Beatmungsschlauch 9 mit einem Patienteninterface 19 und zumindest einem Atemgassensor 11. Das Beatmungsgerät ist ein klinisches Beatmungsgerät oder ein Beatmungsgerät für die häusliche Beatmung oder CPAP- oder Bilevel-Therapie oder ein Highflow-Beatmungsgerät oder ein beliebiges anderes Beatmungsgerät.

Das Beatmungsgerät ist eingerichtet und ausgebildet mittels des Atemgassensors 11 Phasen der Inspiration und Exspiration zu identifizieren und Atemgas während der Inspiration und Exspiration zu fördern und wobei das System eingerichtet ist, zumindest phasenweise während der Inspiration und Exspiration mittels des Sensors 2 und der Verarbeitungseinheit 4 ein Sensorsignal zu ermitteln und zu überprüfen, ob das Sensorsignal repräsentativ für die Körpertemperatur ist und solche Körpertemperatursignale zu verarbeiten und/oder zu speichern und mit hinterlegen Regeln oder Schwellwerten zu vergleichen.
Beatmungsgerät 1
Sensor oder Temperatursensor 2, Körper
Schnittstelle 3 zwischen Beatmungsgerät und Sensor
Verarbeitungseinheit 4
Datenspeicher 5
User Interface 6
Schnittstelle 7 zum Remote Alarm
Remote Alarm 8
Beatmungsschlauch 9 mit einem Patienteninterface 19
Atemgassensor 11
Inspiration 12
Exspiration 13
Aktivitäts-Sensor 14
Anfeuchter 15
Benutzeroberfläche 16
Beatmungsparameter 17
Alarmschwellen 18
Patienteninterface 19
Temperatursensor 20, Umgebung

## Patentansprüche

1. System (10) zur nichtinvasiven Infektionserkennung während der Beatmung mit zumindest einem Sensor (2), welcher zur Infektionserkennung ausgebildet ist, einem Beatmungsgerät (1), einer Schnittstelle (3) zwischen Beatmungsgerät und Sensor (2), einer Verarbeitungseinheit (4), einem Datenspeicher (5), einem User Interface (6), einer Schnittstelle (7) zu einem Remote Alarm, einem Remote Alarm (8), einem Beatmungsschlauch (9) mit einem Patienteninterface (19) und zumindest einem Atemgassensor (11), wobei das Beatmungsgerät eingerichtet und ausgebildet ist mittels des Atemgassensors (11) den Druck und/oder Fluss des Atemgases zu identifizieren und unter Druck stehendes Atemgas zu fördern, wobei das Beatmungsgerät (1) dazu ausgebildet ist eine Atemfrequenz zu bestimmen und wobei das System eingerichtet ist zumindest phasenweise während der Beatmung mittels des Sensors (2) und der Verarbeitungseinheit (4) ein Sensorsignal zu ermitteln und zu überprüfen, ob das Sensorsignal repräsentativ für eine Infektion ist **dadurch gekennzeichnet, dass** die Atemfrequenz mit zumindest dem Sensorsignal verknüpft wird und anhand dieser verknüpften Sensorsignale eine mögliche Infektion beziehungsweise die Wahrscheinlichkeit einer Infektion ermittelt wird.

2. System nach Anspruch 1 wobei der Sensor (2) ein Temperatursensor (2) ist, der einen Anstieg der Körpertemperatur als Infektion erkennt, wenn bestimmte Schwellwerte erreicht oder überschritten sind.

3. System nach Anspruch 2, wobei mit dem zumindest einen Temperatursensor (2) eine Sendeeinrichtung zum Übertragen der gemessenen Temperaturwerte verbunden ist.

4. System nach zumindest einem der vorhergehenden Ansprüche, wobei zumindest ein Temperatursensor (2) oder Sensor (2) in dem Schlauch oder dem Patienteninterface zur Messung der Temperatur der Inspirations- und/oder Exspirationsströmung vorgesehen ist.

5. System nach zumindest einem der vorhergehenden Ansprüche, wobei zumindest ein Temperatursensor (2) oder Sensor (2) dem Körper oder Haut des Patienten benachbart angeordnet ist

6. System nach zumindest einem der vorhergehenden Ansprüche, wobei ergänzend zu dem Temperatursensor (2) oder Sensor (2) auch ein Aktivitäts-Sensor (14) vorgesehen ist.

7. System nach zumindest einem der vorhergehenden Ansprüche, wobei die Verarbeitungseinheit (4) ausgebildet und eingerichtet ist zu erkennen ob ein Sensorsignal vorhanden ist und ob ein Sensorsignal ein Temperatursignal repräsentiert und die Verarbeitungseinheit (4) ausgebildet und eingerichtet ist, das Temperatursignal aufzubereiten beispielsweise durch Umrechnung der Einheiten, Glätten und in dem Datenspeicher (6) abzuspeichern.

8. System nach zumindest einem der vorhergehenden Ansprüche, wobei die Verarbeitungseinheit (4) ausgebildet und eingerichtet ist das Temperatursignal mit hinterlegten Alarmschwellen abzugleichen wobei die Alarmschwellen eine zu hohe Temperatur oder niedrige Temperatur oder den Verlust der Datenverbindung oder ein Abfallen des Sensors oder einen Ablauf der Lebensdauer des Sensors oder zu niedrigem Batterie-Stand des Sensors repräsentieren und, dass die Verarbeitungseinheit (4) ausgebildet und eingerichtet das Temperatursignal mit hinterlegten Alarmschwellen abzugleichen und bei Überschreiten einer Alarmschwelle für erhöhte Körpertemperatur die Verarbeitungseinheit (4) ausgebildet und eingerichtet ist zumindest einen Beatmungsparameter (17) des Beatmungsgerätes so zu steuern, dass eine Beatmung resultiert, die der erhöhten Körpertemperatur angepasst ist, sodass eine optimierte Entlastung oder Unterstützung für den Patient gewährleistet ist.

9. System nach zumindest einem der vorhergehenden Ansprüche, wobei die Verarbeitungseinheit (4) ausgebildet und eingerichtet ist das Temperatursignal mit hinterlegten Alarmschwellen abzugleichen und bei Überschreiten einer Alarmschwelle für eine erhöhte oder erniedrigte Körpertemperatur die Verarbeitungseinheit (4) ausgebildet und eingerichtet ist automatisiert eine Rückmeldung des Patienten anzufordern, wobei die Rückmeldung eine digitale Antwort des Patienten ist, die eine aktuelle Befindlichkeit repräsentiert.

10. System nach zumindest einem der vorhergehenden Ansprüche, wobei die Verarbeitungseinheit (4) ausgebildet und eingerichtet ist das Temperatursignal mit hinterlegten Alarmschwellen abzugleichen und bei Überschreiten einer Alarmschwelle für eine erhöhte oder erniedrigte Körpertemperatur die Verarbeitungseinheit (4) ausgebildet und eingerichtet ist über die Benutzeroberfläche oder einen Lautsprecher Handlungsempfehlungen an den Patienten auszugeben.

11. System nach zumindest einem der vorhergehenden Ansprüche, wobei die Verarbeitungseinheit (4) ausgebildet und eingerichtet ist Signale des Sensors (2) und Signale des Atemgassensors (11) und Signale des Aktivitäts-Sensors (14) zu verarbeiten und Signale des Sensors (2) zu plausibilisieren durch einen Vergleich mit Signalen des Atemgassensors (11) und/oder Signalen des Aktivitäts-Sensors (14).

12. System nach zumindest einem der vorhergehenden Ansprüche, wobei der Datenspeicher (5) ausgebildet und eingerichtet ist den Verlauf des Sensorsignals und vorzugsweise auch die berechneten Statistiken und aufgetretenen Alarme abrufbar aufzuzeichnen.

13. System nach zumindest einem der vorhergehenden Ansprüche, wobei das User Interface (6) ausgebildet und eingerichtet ist ein Rückmeldung zu generieren, ob ein Sensor korrekt angeschlossen ist und Werte liefert oder nicht, bevorzugt in Form einer grafischen und akustischen Ausgabe, z. B. Warnton im Falle eines Alarms.

14. System nach Anspruch 2, wobei das System einen Anfeuchter (15) umfasst, der eingerichtet und ausgebildet ist Atemgas zu erwärmen und anzufeuchten, wobei Körpertemperatursignale des Temperatursensors (2) zumindest zeitweise oder teilweise für die Steuerung des Anfeuchters berücksichtigt werden, um das Atemgas entsprechend dem Köpertemperatursignal zu erwärmen und anzufeuchten.

## Claims

1. A system (10) for non-invasive infection detection during ventilation, comprising at least one sensor (2), which is designed to detect infection, a ventilator (1), an interface (3) between the ventilator and the sensor (2), a processing unit (4), a data memory (5), a user interface (6), an interface (7) to a remote alarm, a remote alarm (8), a ventilation tube (9) having a patient interface (19) and at least one respiratory gas sensor (11), wherein the ventilator is configured and designed to identify the pressure and/or flow of the respiratory gas by means of the respiratory gas sensor (11) and to convey pressurized respiratory gas, wherein the ventilator (1) is designed to determine a breathing rate and wherein the system is configured to ascertain a sensor signal at least in phases during ventilation by means of the sensor (2) and the processing unit (4) and to check whether the sensor signal is representative of an infection, **characterized in that** the breathing rate is linked to at least the sensor signal, and a possible infection or the probability of an infection is ascertained on the basis of these linked sensor signals.

2. The system according to claim 1, wherein the sensor (2) is a temperature sensor (2), which detects an increase in body temperature as an infection when certain threshold values are reached or exceeded.

3. The system according to claim 2, wherein a transmission apparatus for transmitting the measured temperature values is connected to the at least one temperature sensor (2).

4. The system according to at least one of the preceding claims, wherein at least one temperature sensor (2) or sensor (2) is provided in the tube or the patient interface for measuring the temperature of the inspiration and/or expiration flow.

5. The system according to at least one of the preceding claims, wherein at least one temperature sensor (2) or sensor (2) is arranged adjacent to the body or skin of the patient.

6. The system according to at least one of the preceding claims, wherein an activity sensor (14) is also provided in addition to the temperature sensor (2) or sensor (2).

7. The system according to at least one of the preceding claims, wherein the processing unit (4) is designed and configured to detect whether a sensor signal is present and whether a sensor signal represents a temperature signal, and the processing unit (4) is designed and configured to process the temperature signal, for example by converting the units and smoothing, and storing said temperature signal in the data memory (6).

8. The system according to at least one of the preceding claims, wherein the processing unit (4) is designed and configured to compare the temperature signal with stored alarm thresholds, wherein the alarm thresholds represent an excessively high temperature or low temperature or the loss of the data connection or a drop-off of the sensor or an expiry of the service life of the sensor or an excessively low battery level of the sensor, and the processing unit (4) is designed and configured to compare the temperature signal with stored alarm thresholds, and, if an alarm threshold for a raised body temperature is exceeded, the processing unit (4) is designed and configured to control at least one ventilation parameter (17) of the ventilator in such a way that ventilation results which is adjusted to the raised body temperature so that optimized alleviation or support for the patient is ensured.

9. The system according to at least one of the preceding claims, wherein
the processing unit (4) is designed and configured to compare the temperature signal with stored alarm thresholds, and, if an alarm threshold for a raised or lowered body temperature is exceeded, the processing unit (4) is designed and configured to request feedback from the patient in an automated manner, wherein the feedback is a digital response of the patient, which represents a current state.

10. The system according to at least one of the preceding claims, wherein
the processing unit (4) is designed and configured to compare the temperature signal with stored alarm thresholds, and, if an alarm threshold for a raised or lowered body temperature is exceeded, the processing unit (4) is designed and configured to output recommendations for action via the user interface or a speaker to the patient.

11. The system according to at least one of the preceding claims, wherein the processing unit (4) is designed and configured to process signals from the sensor (2) and signals from the respiratory gas sensor (11) and signals from the activity sensor (14) and to check signals from the sensor (2) for plausibility by comparing with signals from the respiratory gas sensor (11) and/or signals from the activity sensor (14).

12. The system according to at least one of the preceding claims, wherein the data memory (5) is designed and configured to retrievably record the profile of the sensor signal and preferably also the calculated statistics and alarms which have occurred.

13. The system according to at least one of the preceding claims, wherein the user interface (6) is designed and configured to generate feedback as to whether a sensor is correctly connected and is providing values or not, preferably in the form of a graphical and acoustic output, for example a warning sound in the case of an alarm.

14. The system according to claim 2, wherein the system comprises a humidifier (15), which is configured and designed to heat and moisten respiratory gas, wherein body temperature signals of the temperature sensor (2) are taken into account at least at times or partially for controlling the humidifier in order to heat and moisten the respiratory gas in accordance with the body temperature signal.

## Revendications

1. Système (10) destiné à la détection non invasive d'une infection lors de la respiration avec au moins un capteur (2), lequel est conçu pour la détection d'infection, un appareil respiratoire (1), une interface (3) entre l'appareil respiratoire et le capteur (2), une unité de traitement (4), une mémoire de données (5), une interface utilisateur (6), une interface (7) vers une alarme à distance, une alarme à distance (8), un tube respiratoire (9) avec une interface de patient (19) et au moins un capteur de gaz respiratoire (11), l'appareil respiratoire étant configuré et conçu pour identifier la pression et/ou le flux du gaz respiratoire au moyen du capteur de gaz respiratoire (11) et pour transporter du gaz respiratoire sous pression, l'appareil respiratoire (1) étant conçu pour déterminer une fréquence respiratoire et le système étant configuré pour détecter un signal de capteur pendant la respiration au moyen du capteur (2) et de l'unité de traitement (4), au moins par phases, et pour vérifier si le signal de capteur est représentatif pour une infection, **caractérisé en ce que** la fréquence respiratoire est associée au moins avec le signal de capteur et **en ce qu'**une infection éventuelle ou la probabilité d'une infection est détectée à l'aide de ces signaux de capteur associés.

2. Système selon la revendication 1, le capteur (2) étant un capteur de température (2), lequel détecte comme infection une hausse de la température du corps, lorsque des valeurs seuils définies sont atteintes ou dépassées.

3. Système selon la revendication 2, un dispositif émetteur étant connecté avec l'au moins un capteur de température (2) pour le transfert des valeurs de température mesurées.

4. Système selon au moins l'une des revendications précédentes, au moins un capteur de température (2) ou capteur (2) étant prévu dans le tuyau ou dans l'interface de patient pour la mesure de la température du flux inspiratoire et/ou expiratoire.

5. Système selon au moins l'une des revendications précédentes, au moins un capteur de température (2) ou capteur (2) étant disposé à proximité du corps ou de la peau du patient.

6. Système selon au moins l'une des revendications précédentes, un capteur d'activité (14) étant également prévu en complément du capteur de température (2) ou capteur (2).

7. Système selon au moins l'une des revendications précédentes, l'unité de traitement (4) étant conçue et configurée pour détecter si un signal de capteur est présent et si un signal de capteur représente un signal de température, et l'unité de traitement (4) étant conçue et configurée pour préparer le signal de température, par exemple par conversion des unités, lissage et enregistrement dans la mémoire de données (6).

8. Système selon au moins l'une des revendications précédentes, l'unité de traitement (4) étant conçue et configurée pour comparer le signal de température avec des seuils d'alarme enregistrés, les seuils d'alarme représentant une température trop élevée ou une température trop basse ou la perte de la connexion de données ou une baisse du capteur ou une expiration de la durée de vie du capteur ou un niveau de batterie trop bas du capteur, et l'unité de traitement (4) étant conçue et configurée pour comparer le signal de température avec les seuils d'alarme enregistrés et, lors du l'unité de traitement (4) étant conçue et configurée, lors du dépassement d'un seuil d'alarme pour température du corps élevée, pour contrôler au moins un paramètre de respiration (17) de l'appareil respiratoire de telle sorte qu'une respiration est effectuée, laquelle est ajustée à la température du corps élevée, de telle sorte qu'un soutien ou soulagement optimisé pour le patient est garanti.

9. Système selon au moins l'une des revendications précédentes, l'unité de traitement (4) étant conçue et configurée pour comparer le pour comparer le signal de température avec les seuils d'alarme enregistrés et, lors du dépassement d'un seuil d'alarme pour une température du corps élevée ou basse, l'unité de traitement (4) étant conçue et configurée pour demander de façon automatisée un retour du patient, le retour étant une réponse numérique du patient, laquelle représente un état actuel.

10. Système selon au moins l'une des revendications précédentes, l'unité de traitement (4) étant conçue et configurée pour comparer le signal de température avec les seuils d'alarme enregistrés et, lors du dépassement d'un seuil d'alarme pour une température du corps élevée ou basse, l'unité de traitement (4) étant conçue et configurée pour émettre à l'attention du patient des recommandations d'action par l'interface utilisateur ou par un haut-parleur.

11. Système selon au moins l'une des revendications précédentes, l'unité de traitement (4) étant conçue et configurée pour traiter des signaux du capteur (2) et des signaux du capteur de gaz respiratoire (11) et des signaux du capteur d'activité (14), et pour plausibiliser des signaux du capteur (2) par une comparaison avec des signaux du capteur de gaz respiratoire (11) et/ou des signaux du capteur d'activité (14).

12. Système selon au moins l'une des revendications précédentes, la mémoire de données (5) étant conçue et configurée pour enregistrer de façon consultable l'allure du signal de capteur et, de préférence, également les statistiques calculées et des alarmes apparues.

13. Système selon au moins l'une des revendications précédentes, l'interface utilisateur (6) étant conçue et configurée pour générer un retour relativement au fait de savoir si un capteur est relié correctement et si des valeurs sont fournies ou non, de préférence sous la forme d'une sortie graphique et acoustique, par exemple un avertissement sonore dans le cas d'une alarme.

14. Système selon la revendication 2, le système comprenant un humidificateur (15), lequel est configuré et conçu pour chauffer et humidifier du gaz respiratoire, les signaux de température du corps du capteur de température (2) étant pris en compte au moins temporairement ou partiellement pour le contrôle de l'humidificateur, afin de chauffer et humidifier le gaz respiratoire d'une façon correspondant au signal de température du corps.
